# EUROPEAN PATENT SPECIFICATION

(11) **EP 2 694 473 B1**
(45) Date of publication and mention of the grant of the patent: **02.01.2019**
(21) Application number: 12706857.5
(22) Date of filing: 05.03.2012
(51) Int. Cl.: C09B 29/30, C09D 11/328, C09B 62/51, D06P 5/30, C09B 62/475, C09B 62/513, C09B 62/44, D06P 3/66, C07C 317/44, D06P 1/384

(54) **FIBRE-REACTIVE DYES, THEIR PREPARATION AND THEIR USE**
REAKTIVE FASERFARBSTOFFE SOWIE IHRE HERSTELLUNG UND VERWENDUNG
COLORANTS RÉACTIFS, LEUR PRÉPARATION ET LEUR UTILISATION

(30) Priority: 07.04.2011 EP 11161503
(43) Date of publication of application: 12.02.2014
(73) Proprietor: Huntsman Advanced Materials (Switzerland) GmbH, 4057 Basel (CH)
(72) Inventor: ROENTGEN, Georg, 79108 Freiburg (DE); FEKETE, Laszlo, 4312 Magden (CH); NICOLLET, Michael, 68128 Village-Neuf (FR)
(86) International application number: PCT/EP2012/053724
(87) International publication number: WO 2012/136428

(56) References cited:
- EP-A1- 1 041 121
- WO-A2-00/06652
- WO-A2-2007/003541
- US-B1- 6 392 021

## Description

The present invention relates to fibre-reactive dyes, to a process for their preparation and to their use in the dyeing or printing of textile fibre materials.

The practice of dyeing using reactive dyes has recently led to higher demands being made on the quality of the dyeings and the economic efficiency of the dyeing process. As a result, there continues to be a need for novel reactive dyes having improved properties, especially in respect of their application.

Dyeing nowadays requires reactive dyes that have sufficient substantivity and at the same time have good ease of washing off of unfixed dye. They should also have a good colour yield and high reactivity, the objective being to provide especially dyeings having high degrees of fixing. The known dyes do not satisfy these requirements in all properties.

The dyes known from JP-A-08-60017 and US-A-6,537,332 still have certain disadvantages in respect of the above-mentioned properties.

The problem underlying the present invention is accordingly to find, for the dyeing and printing of fibre materials, novel improved reactive dyes having the qualities characterised above to a high degree. The novel dyes should especially be distinguished by good build-up properties, high fixing yields and high fibre-dye bond stabilities, and in addition it should be possible for dye not fixed to the fibre to be washed off readily. The dyes should also yield dyeings having good allround fastness properties, for example fastness to light and wash fastness.

It has been found that the problem posed is largely solved by the novel dyes defined hereinbelow.

The present invention therefore relates to reactive dyes of the formula 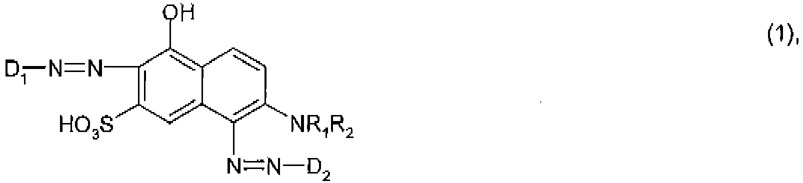 wherein
R₁ is hydrogen,
R₂ is hydrogen, methyl or a radical of the formula -CH₂-SO₃H,
D₁ corresponds to a radical of the formula 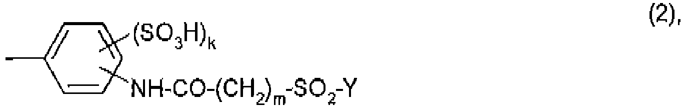 and
D₂ is a radical of formula 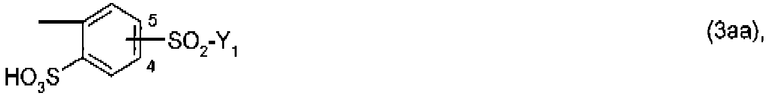
wherein
   Y₁ is vinyl or β-sulfatoethyl.
   Y is vinyl, β-chloroethyl or β-sulfatoethyl,
   k is the number 1 or 2, and
   m is the number 2 or 3, preferably 3.

As C₁-C₄alkyl there comes into consideration for R₂, for example, methyl, ethyl, n-propyl, isopropyl, n-butyl, sec-butyl, isobutyl and tert-butyl, especially methyl and ethyl. The alkyl radicals R₂ may be unsubstituted or may be substituted, for example, by hydroxy, sulfo, sulfato, cyano, carboxy, C₁-C₄alkoxy or by phenyl, preferably by hydroxy, sulfo, C₁-C₄alkoxy or by phenyl, and especially by sulfo.

In a preferred embodiment R₂ is hydrogen.

m is especially preferably the number 3.

The present invention relates also to a process for the preparation of dyes of the formula (1). which comprises
(i) diazotisation of approximately one molar equivalent of an amine of the formula

   D₂-NH₂ (6)

   in customary manner and coupling reaction with approximately one molar equivalent of a compound of the formula 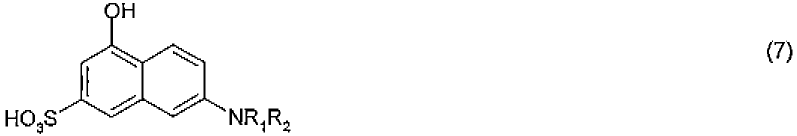 (first coupling reaction) to form a compound of formula 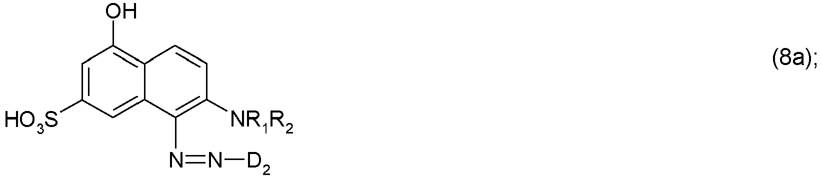 and
(ii) diazotisation of approximately one molar equivalent of an amine of the formula

   D₁-NH₂ (9)

   in customary manner and coupling reaction with approximately one molar equivalent of the compound of the formula (8a) obtained according to (i) to form a compound of the formula (1), (second coupling reaction), wherein D₁, D₂, R₁ and R₂ each have the definitions and preferred meanings given hereinabove.

The diazotisation of the amines of formulae (6) and (9) is carried out in a manner known *per se*, for example using a nitrite, for example an alkali metal nitrite such as sodium nitrite, in a mineral acid medium, for example in a hydrochloric acid medium, at temperatures of, for example, from -5 to 40°C and preferably at from 0 to 20°C.

The coupling to the coupling components of formulae (7) and (8a) is carried out in a manner known *per se* at acidic or neutral to slightly alkaline pH values, for example a pH value of from 0 to 8, and at temperatures of, for example, from -5 to 40°C, preferably from 0 to 30°C.

The first coupling reaction - (i) - takes place in an acidic medium, for example at a pH of from 0 to 4, and the second coupling reaction - (ii) - at elevated pH values, in a slightly acidic, neutral or slightly alkaline medium, for example a pH value of from 4 to 8.

By proceeding as described hereinabove but, instead of using approximately one molar equivalent each of the amines of formulae (6) and (9) in process steps (i) and (ii), using in each case approximately one molar equivalent of a mixture of at least two, preferably two, non-identical amines, for example a 1:1 molar mixture of the compounds of formulae (6) and (9), there is obtained first of all, according to (i), a mixture of compounds of formulae 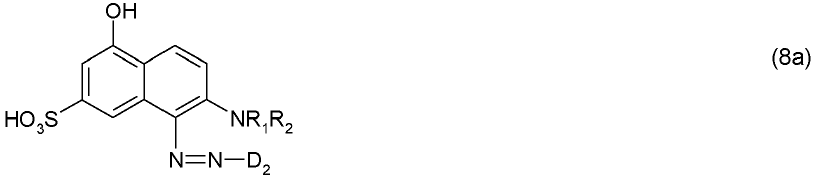 and 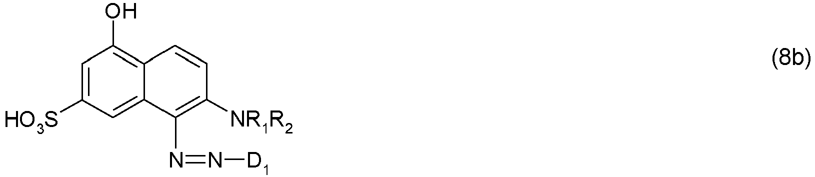 and, on further reaction of the mixture of compounds of formulae (8a) and (8b) according to (ii), a mixture of dyes of formulae (1), (1b), (1c) and (1d) 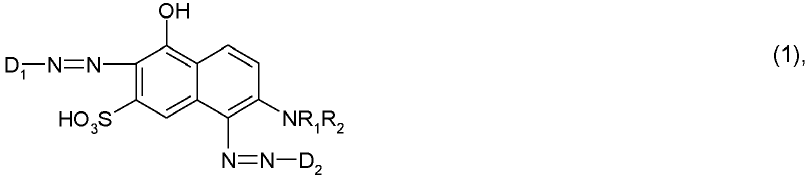 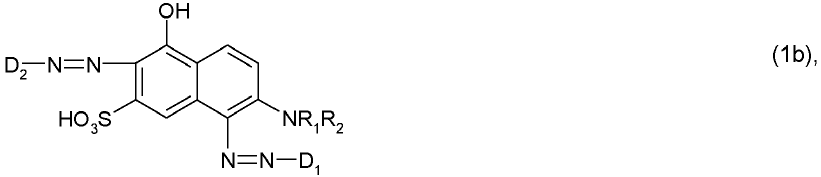 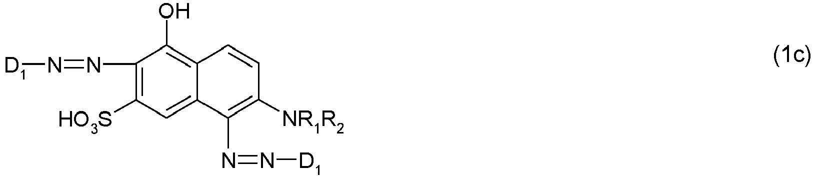 and

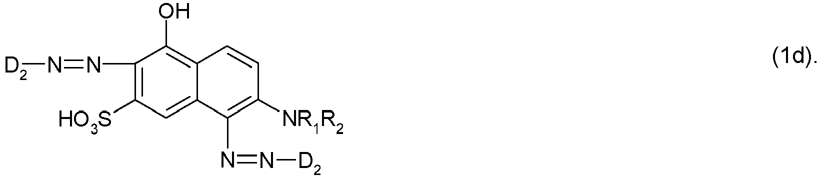

The present invention accordingly relates also to dye mixtures that comprise at least one dye of formulae (1) and (1b) together with at least one dye of formulae (1c) and (1d), especially one dye each of formulae (1), (1b), (1c) and (1d), wherein D₁, D₂, R₁ and R₂ each have the definitions and preferred meanings given hereinabove.

The ratio of the dyes of formulae (1), (1b), (1c) and (1d) in the mixture can vary within wide limits and depends on the ratio of the particular amines D₁-NH₂ and D₂-NH₂ used according to (i) and (ii).

The above dye mixtures contain, for example, from 5 to 95 % by weight, especially from 10 to 90 % by weight and preferably from 20 to 80 % by weight, of a dye of formula (1) and/or (1b), based on the total amount of the dyes of formulae (1), (1b), (1c) and (1d) in the mixture.

The compounds of formulae (6), (7) and (9) are known or can be obtained in a manner known *per se.*

For example, the compound of formula (6) wherein D₂ is a radical of the above formula (3a) with Z₁ in the meaning of a radical of forumula (4e) can be prepared by condensation of approximately one molar equivalent of 2,4,6-trichloro-s-triazine or 2,4,6-trifluoro-s-triazine first with approximately one molar equivalent of a compound of formula

T₁-H (10),

at a pH value in the neutral range and at low temperature, for example from 0 to 5°C.

Such condensation reactions are known and are described, for example, in EP-A-0 260 227 and US-A-4 841 049.

Some of the compounds of the formula (9) are new. Therefore, the present invention also relates to compounds of the formula 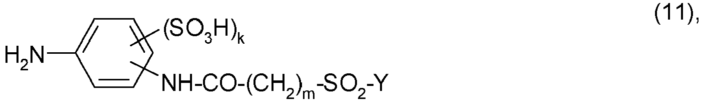 wherein
Y is vinyl or a -CH₂-CH₂-U radical and U is a group that is removable under alkaline conditions,
k is the number 2, and
m is the number 3,
wherein Y has the definitions and preferred meanings given hereinabove.

The preparation of the compounds of the formula (11) is described in the experimental part.

The reactive dyes of the formula (1), the dye mixtures comprising at least one dye of formulae (1) and (1b) together with at least one dye of formulae (1c) and (1d), and the compounds of formula (11) according to the present invention are either in the form of their free acids or, preferably, in the form of salts thereof. Salts that come into consideration are, for example, alkali metal, alkaline earth metal and ammonium salts, and salts of an organic amine. Sodium, lithium, potassium and ammonium salts and the salt of the mono-, di- or triethanolamine may be mentioned as examples.

If appropriate, the end product can also be subjected to a conversion reaction. Such a conversion reaction is, for example, the conversion of a vinylatable reactive group contained in Z₁ or Y into its vinyl form by treatment with dilute sodium hydroxide solution, for example the conversion of the β-sulfatoethylsulfonyl or β-chloroethylsulfonyl group into the vinylsulfonyl radical or the conversion of the α,β-dihalopropionylamino group into the α-haloacryloylamino radical. Such reactions are known *per se.* The conversion reaction is generally carried out in neutral to alkaline medium at a temperature of, for example, from 20 to 70°C, at a pH value of, for example, from 6 to 14.

The dyes and the dye mixtures according to the present invention are fibre-reactive. Fibre-reactive dyes are to be understood as being those which are capable of reacting with the hydroxyl groups of cellulose, with the amino, carboxy, hydroxy or thiol groups in wool and silk or with the amino and possibly carboxy groups of synthetic polyamides to form covalent chemical bonds.

The dyes and the dye mixtures according to the invention are suitable for dyeing and printing an extremely wide variety of materials, such as hydroxyl-group-containing or nitrogen-containing fibre materials. Examples are silk, leather, wool, polyamide fibres and polyurethanes and also especially cellulosic fibre materials of all kinds. Such cellulosic fibre materials are, for example, the natural cellulosic fibres, such as cotton, linen and hemp, and also cellulose and regenerated cellulose. The dyes and the dye mixtures according to the invention are also suitable for dyeing or printing hydroxyl-group-containing fibres present in blend fabrics, e.g. mixtures of cotton with polyester fibres or polyamide fibres. The dyes and the dye mixtures according to the invention are especially suitable for dyeing or printing cellulosic, especially cotton-containing, fibre materials. They may also be used for dyeing or printing natural or synthetic polyamide fibre materials.

The dyes and the dye mixtures according to the invention can be applied to the fibre material and fixed to the fibre in a variety of ways, especially in the form of aqueous dye solutions and dye print pastes. They are suitable both for the exhaust process and for dyeing in accordance with the pad-dyeing process, according to which the goods are impregnated with aqueous, optionally salt-containing, dye solutions and the dyes are fixed, after treatment with an alkali or in the presence of an alkali, optionally under the action of heat or as a result of being kept at room temperature for several hours. After fixing, the dyeings or prints are thoroughly rinsed with cold and hot water, optionally with addition of an agent that has a dispersing action and promotes diffusion of unfixed dye.

The dyes and the dye mixtures according to the invention are distinguished by high reactivity, good fixing capacity and very good build-up capacity. They can therefore be used in the exhaust dyeing process at low dyeing temperatures and require only short steaming times in the pad-steam process. The degrees of fixing are high and unfixed dye can be washed off readily, the difference between the degree of exhaust and the degree of fixing being remarkably small, that is to say the soaping loss is very small. The dyes and the dye mixtures according to the invention are also especially suitable for printing, more especially on cotton, and also for printing nitrogen-containing fibres, for example wool or silk or blend fabrics that contain wool or silk.

The dyeings and prints produced using the dyes according to the invention have a high tinctorial strength and a high fibre-dye binding stability in both the acidic and the alkaline range, as well as good fastness to light and very good wet-fastness properties, such as fastness to washing, to water, to seawater, to cross-dyeing and to perspiration, and also good fastness to pleating, to hot pressing and to rubbing.

The reactive dyes of the formula (1), and the dye mixtures comprising at least one dye of formulae (1) and (1b) together with at least one dye of formulae (1c) and (1d) according to the present invention can advantageously be applied in combination with other reactive dyes, for example, navy, dark blue or black dyeing dyestuffs such as C.I. Reactive Black 5, or the dyes described in US-A-4,622,390, US-A-6,458,936 and WO 2004/088031 (dyes of formulae (102), (104), (105), (106) and (107)) which documents are incorporated hereby as a reference.

The present invention relates also to aqueous inks comprising a reactive dye of the formula (1), the respective definitions and preferred meanings given above applying for D₁, D₂, R₁ and R₂.

The dyes used in the inks should preferably have a low salt content, that is to say they should have a total content of salts of less than 0.5 % by weight, based on the weight of the dyes. Dyes that have relatively high salt contents as a result of their preparation and/or as a result of the subsequent addition of diluents can be desalted, for example, by membrane separation procedures, such as ultrafiltration, reverse osmosis or dialysis.

The inks preferably have a total content of dyes of from 1 to 35 % by weight, especially from 1 to 30 % by weight and preferably from 1 to 20 % by weight, based on the total weight of the ink. As a lower limit, a limit of 1.5 % by weight, preferably 2 % by weight and especially 3 % by weight, is preferred.

The inks may comprise water-miscible organic solvents, for example C₁-C₄alcohols, such as methanol, ethanol, n-propanol, isopropanol, n-butanol, sec-butanol, tert-butanol and isobutanol; amides, e.g. dimethylformamide and dimethylacetamide; ketones or ketone alcohols, e.g. acetone and diacetone alcohol; ethers, e.g. tetrahydrofuran and dioxane; nitrogen-containing heterocyclic compounds, e.g. N-methyl-2-pyrrolidone and 1,3-dimethyl-2-imidazolidone; polyalkylene glycols, e.g. polyethylene glycol and polypropylene glycol; C₂-C₆-alkylene glycols and thioglycols, e.g. ethylene glycol, propylene glycol, butylene glycol, triethylene glycol, thiodiglycol, hexylene glycol and diethylene glycol; further polyols, e.g. glycerol and 1,2,6-hexanetriol; and C₁-C₄alkyl ethers of polyhydric alcohols, e.g. 2-methoxyethanol, 2-(2-methoxyethoxy)ethanol, 2-(2-ethoxyethoxy)ethanol, 2-[2-(2-methoxyethoxy)-ethoxy]ethanol and 2-[2-(2-ethoxyethoxy)ethoxy]ethanol; preferably N-methyl-2-pyrrolidone, diethylene glycol, glycerol or especially 1,2-propylene glycol, usually in an amount of from 2 to 30 % by weight, especially from 5 to 30 % by weight and preferably from 10 to 25 % by weight, based on the total weight of the ink.

The inks may also comprise solubilisers, e.g. ε-caprolactam.

The inks may comprise thickeners of natural or synthetic origin *inter alia* for the purpose of adjusting the viscosity.

Examples of thickeners that may be mentioned include commercially available alginate thickeners, starch ethers or locust bean flour ethers, especially sodium alginate on its own or in admixture with modified cellulose, e.g. methylcellulose, ethylcellulose, carboxymethylcellulose, hydroxyethylcellulose, methyl hydroxyethylcellulose, hydroxypropyl cellulose or hydroxypropyl methylcellulose, especially with preferably from 20 to 25 % by weight carboxymethylcellulose. Synthetic thickeners that may be mentioned are, for example, those based on poly(meth)acrylic acids or poly(meth)acrylamides.

The inks comprise such thickeners, for example, in an amount of from 0.01 to 2 % by weight, especially from 0.01 to 1 % by weight and preferably from 0.01 to 0.5 % by weight, based on the total weight of the ink.

The inks may also comprise buffer substances, e.g. borax, borates, phosphates, polyphosphates or citrates. Examples that may be mentioned include borax, sodium borate, sodium tetraborate, sodium dihydrogen phosphate, disodium hydrogen phosphate, sodium tripolyphosphate, sodium pentapolyphosphate and sodium citrate. They are used especially in amounts of from 0.1 to 3 % by weight, preferably from 0.1 to 1 % by weight, based on the total weight of the ink, in order to establish a pH value of, for example, from 4 to 9, especially from 5 to 8.5.

As further additives, the inks may comprise surfactants or humectants.

Suitable surfactants include commercially available anionic or non-ionic surfactants. As humectants in the inks according to the invention there come into consideration, for example, urea or a mixture of sodium lactate (advantageously in the form of a 50 % to 60 % aqueous solution) and glycerol and/or propylene glycol in amounts of preferably from 0.1 to 30% by weight, especially from 2 to 30 % by weight.

Preference is given to inks having a viscosity of from 1 to 40 mPa·s, especially from 1 to 20 mPa·s and preferably from 1 to 10 mPa·s.

The inks may also comprise customary additives, such as antifoam agents or especially substances that inhibit the growth of fungi and/or bacteria. Such additives are usually used in amounts of from 0.01 to 1 % by weight, based on the total weight of the ink.

The inks can be prepared in customary manner by mixing together the individual constituents in the desired amount of water.

The inks according to the invention are especially suitable for use in recording systems of a kind wherein an ink is expressed from a small opening in the form of droplets that are directed towards a substrate on which an image is produced. Suitable substrates are, for example, paper, textile fibre materials or plastics films. Suitable recording systems are, for example, commercially available inkjet printers for use in paper or textile printing, or writing instruments, such as fountain pens or ballpoint pens and especially inkjet printers.

Depending on the nature of its use, it may be necessary for e.g. the viscosity or other physical properties of the ink, especially those properties affecting the affinity for the substrate in question, to be modified as appropriate.

As examples of paper that can be printed with the inks according to the invention there may be mentioned commercially available inkjet paper, photo paper, glossy paper, plastics-coated paper, e.g. Epson inkjet paper, Epson photo paper, Epson glossy paper, Epson glossy film, HP special inkjet paper, Encad photo gloss paper and Ilford photo paper. Plastics films that can be printed with the inks according to the invention are, for example, transparent or cloudy/opaque. Suitable plastics films are, for example, 3M transparency film.

As textile fibre materials there come into consideration, for example, nitrogen-containing or hydroxy-group-containing fibre materials, for example textile fibre materials of cellulose, silk, wool or synthetic polyamides, preferably cellulose.

The present invention accordingly relates also to a method of printing textile fibre materials, paper or plastics films, preferably textile fibre materials or paper and especially textile fibre materials, by the inkjet printing method, wherein an aqueous ink comprising a reactive dye of the formula (1) is used, the respective definitions and preferred meanings given above applying for D₁, D₂, R₁ and R₂.

In the inkjet printing method, individual droplets of the ink are sprayed onto a substrate in a controlled manner from a nozzle. For this purpose, predominantly the continuous inkjet method and the drop-on-demand method are used. In the continuous inkjet method, the droplets are produced continuously and any droplets not required for the printing are conveyed to a collecting vessel and recycled, whereas in the drop-on-demand method droplets are produced and printed as required; that is to say droplets are produced only when required for the printing. The production of the droplets can be effected, for example, by means of a piezo-inkjet head or by means of thermal energy (bubble jet). Preference is given to printing by means of a piezo-inkjet head for the method according to the invention. In addition, preference is given to printing in accordance with the continuous inkjet method for the method according to the invention.

The recordings, e.g. prints, which are produced are distinguished especially by a high degree of colour strength and of colour brilliance and also by good light- and wet-fastness properties.

The following Examples serve to illustrate the invention. Unless otherwise indicated, the temperatures are given in degrees Celsius, parts are parts by weight and percentages relate to % by weight. Parts by weight relate to parts by volume in a ratio of kilograms to litres.

### Example 1:

80.5 g of 1,3-phenylenediamine-4,6-disulfonic acid are dissolved in 300 ml of water at pH 6 to 7 by means of 60 ml of an aqueous sodium hydroxyde solution (30%). Subsequently, water is added to adjust the volume of the mixture to 500 ml. 250 g of crushed ice are introduced, and 189 g of 4-(2-chloroethylsulfonyl)-butyrylchloride are added slowly while vigorously stirring the mixture. During addition, temperature and pH of the reaction mixture are maintained at 0 to 3°C and pH 5 by means of crushed ice and aqueous soda (20% solution).

After completion of the reaction insoluble parts are removed by filtration. The solution obtained is adjusted to pH 1.5 by means of hydrochloric acid (32%). Sodium chloride is added under agitation in order to precipitate the product. The precipitate is separated and suspended in water. Aqueous soda (20%) is added in order to dissolve the precipitate at pH 4. The solution obtained is adjusted to pH 1.5 by means of hydrochloric acid (32%). Sodium chloride is added under agitation in order to precipitate the product. The precipitate is separated and suspended in water. Aqueous soda (20%) is added in order to dissolve the precipitate at pH 6.5. The solution contains as the main product a compound which in the form of the free acid corresponds to the formula

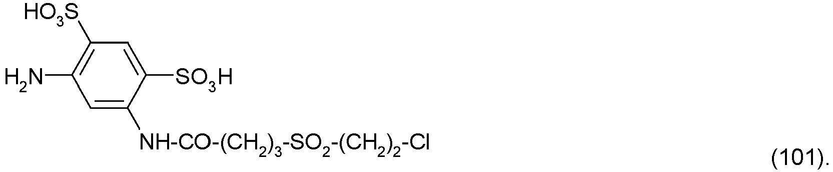

### Example 2:

80.5 g of 1,4-phenylenediamine-2,6-disulfonic acid are dissolved in 300 ml of water at around pH 7 by means of 30 ml of an aqueous sodium hydroxyde solution (30%). The volume of the mixture is adjusted to 400 ml by adding water. 300 g of crushed ice are introduced, and 82 g of 4-(2-chloroethylsulfonyl)-butyrylchloride are added stepwise while vigorously stirring the mixture, and maintaining the pH at 7.0 with aqueous soda (20% solution). During addition, the temperature of the reaction mixture is maintained at 0 to 5°C by means of crushed ice. After completion of the reaction water is added to increase the volume of the mixture to 900 ml. Insoluble parts are separated by filtration. The solution contains as the main product a compound which in the form of the free acid corresponds to the formula

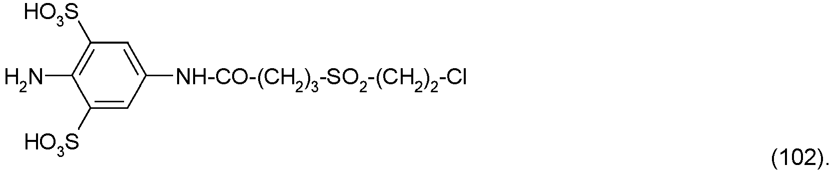

### Example 3:

161 g of 1,4-phenylenediamine-2,5-disulfonic acid are dissolved in 800 ml of water at pH 7 by means of 120 ml of an aqueous sodium hydroxyde solution (30%). Subsequently, 1000 g of crushed ice are added and the mixture is adjusted to pH 4.8 by addition of 20 ml of hydrochloric acid (32%). 245 g of 4-(2-chloroethylsulfonyl)-butyrylchloride are, then, added slowly while vigorously stirring the mixture. During addition, temperature and pH of the reaction mixture are maintained at 0 to 10°C and pH 5 by means of crushed ice and aqueous soda (20% solution).

After completion of the reaction insoluble parts are separated by filtration over Dicalite at approximately 10°C. The solution contains as the main product a compound which in the form of the free acid corresponds to the formula

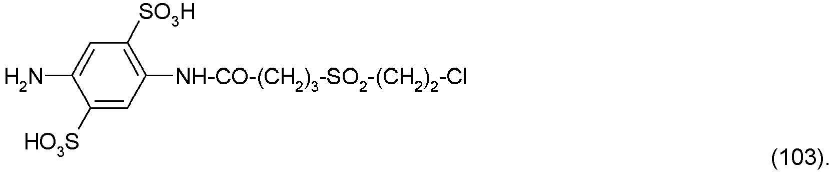

The product can be isolated by dialysis of the solution to remove salt and subsequent concentration by evaporation *in vacuo.*

### Example 4:

(a) Diazotation: 704 g of the solution obtained according to Example 1 are cooled to 10°C and acidified with 15 ml of hydrochloric acid (32%). At 10°C, there are added to the resulting solution 12.5 ml of a 4N sodium nitrite solution. The reaction mixture is stirred for 30 minutes and excess of nitrite is destroyed by the addition of sulfamic acid. A yellow suspension is obtained.
(b) Second coupling: 2.5 g of disodium hydrogen phosphate are introduced into 385 g of an aqueous solution containing approximately 30.6 g of the compound of the formula

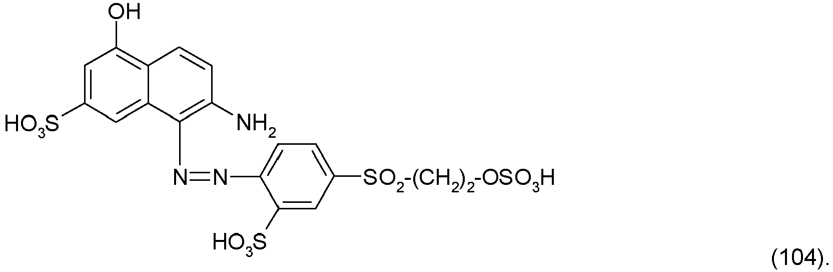

The temperatur of the solution is adjusted to 5°C, and the yellow suspension obtained according to step (a) above is added slowly, while maintaining the mixture at pH 5 by addition of aqueous soda (20% solution). When coupling is complete, the dye solution is freed of salt by dialysis and concentrated by evaporation *in vacuo* to obtain a product which contains mainly a compound that in the form of the free acid corresponds to the formula 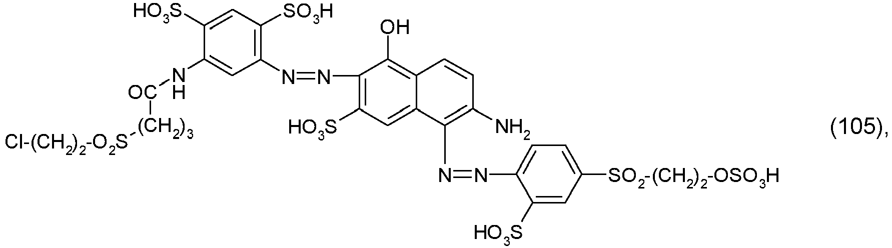 (λₘₐₓ: 502 nm) and dyes cellulose in a reddish shade with good allround properties.

The compound of formula (104) can be obtained according to the process described in Example 21 of US-A-7,572,301 or Example 26 of US-A-6,537,332 (first coupling reaction). Monoazo compounds within the scope of the present invention which are similar to the compound of formula (104) can be obtained in analogy to the process described in US-A-7,572,301 and US-A-6,537,332 (first coupling reaction).

### Example 5:

(a) Diazotation: 382.6 g of the solution obtained according to Example 2 are cooled to 10°C and acidified with 28 ml of hydrochloric acid (32%). At 10°C, there are added to the resulting solution 25 ml of a 4N sodium nitrite solution. The reaction mixture is stirred for 30 minutes and excess of nitrite is destroyed by the addition of sulfamic acid. A yellow suspension is obtained.
(b) Second coupling: 20 g of an aqueous disodium hydrogen phosphate solution (25%) are added to the yellow suspension obtained according to step (a) at a temperature of 5°C. To this stirred and cooled suspension 769.3 g of an aqueous solution containing approximately 61.2 g of the compound of formula (104) are introduced slowly, while maintaining the reaction mixture at pH 5 by addition of aqueous soda (20% solution). Coupling is completed by continued agitation and successively increasing to pH of the reaction mixture to pH 7.5 by further addition of aqueous soda (20% solution). Subsequently, the dyestuff solution obtained is adjusted to pH 6 , freed of salt by dialysis and concentrated by evaporation *in vacuo* to obtain a product which contains mainly a compound that in the form of the free acid corresponds to the formula 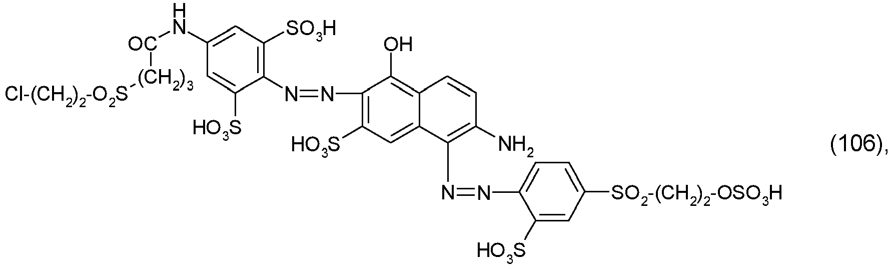 (λₘₐₓ: 490 nm) and dyes cellulose in a red-brown shade with good allround properties.

### Example 6:

(a) Diazotation: 218.2 g of the amine of the formula 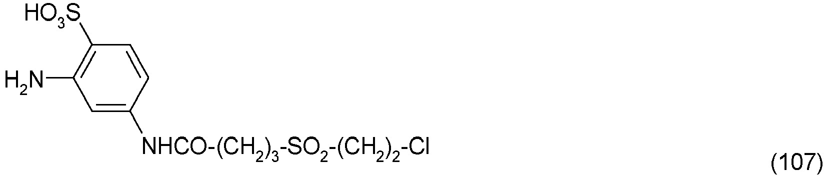 are suspended in 500 ml of water and the volume of the suspension is adjusted to 2800 ml by further addition of water. 196 g of sodium chloride are added and the mixture is acidified with 121 ml of hydrochloric acid (32%). At room temperature, there are added to the resulting solution within 45 minutes 136 ml of a 4N sodium nitrite solution. The suspension obtained is stirred until the reaction is completed and excess of nitrite is destroyed by the addition of sulfamic acid.
(b) Second coupling: 3645 g of an aqueous solution containing approximately 318 g of the compound of formula (104) are adjusted to pH 6.5 by addition of aqueous soda (20% solution). 3192 g of the suspension obtained according to step (a) above are added slowly, while maintaining the mixture at pH 6.5 by addition of aqueous soda (20% solution). Coupling is completed over night. The reaction mixture is adjusted to pH 5.5 by means of hydrochloric acid (32%). Subsequently, the dye solution is freed of salt by dialysis and concentrated by evaporation *in vacuo* to obtain a product which contains mainly a compound that in the form of the free acid corresponds to the formula 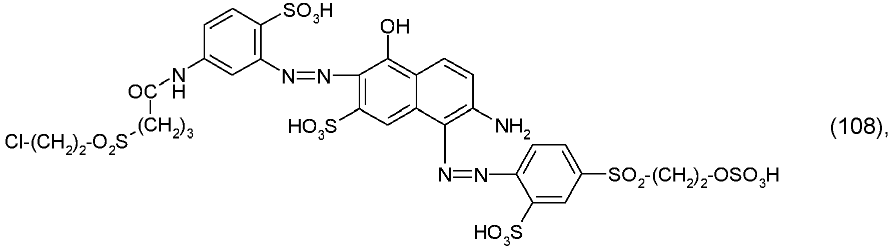 (λₘₐₓ: 506 nm) and dyes cellulose in a red shade with good allround properties.

### Example 7:

(a) Diazotation: 26.3 g of the isolated product obtained according to Example 3 are suspended in 200 ml of water. The mixture is acidified with 10 ml of hydrochloric acid (32%). At temperature of from 15-25°C, there are added to the resulting solution within 40 minutes 8.2 ml of a 4N sodium nitrite solution. The suspension obtained is stirred until the reaction is completed and excess of nitrite is destroyed by the addition of sulfamic acid.
(b) Second coupling: 298 g of an aqueous solution containing approximately 20.1 g of a compound of the formula (104) are adjusted to pH 6.5 by addition of aqueous soda (20% solution). The suspension obtained according to step (a) above is added slowly, while maintaining the mixture at from pH 6.5-7.5 by addition of aqueous soda (20% solution). The reaction mixture is adjusted to pH 5.5 by means of hydrochloric acid (32%) and coupling is completed over night. Subsequently, the dye solution is freed of salt by dialysis and concentrated by evaporation *in vacuo* to obtain 50.2 g of a product which contains mainly a compound that in the form of the free acid corresponds to the formula 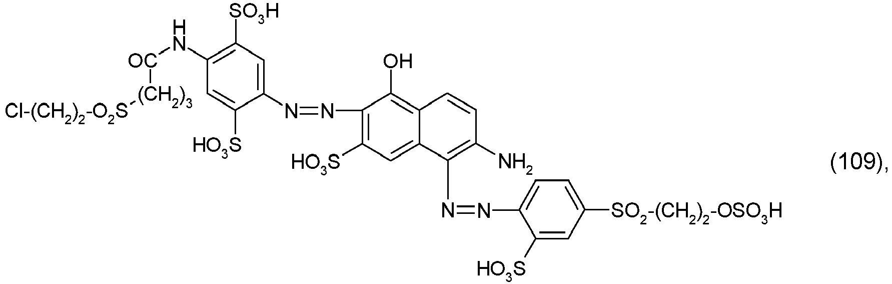 (λₘₐₓ: 514 nm) and dyes cellulose in a red shade with good allround properties.

### Example 8:

(a) Diazotation: 9.6 g of the amine of the formula 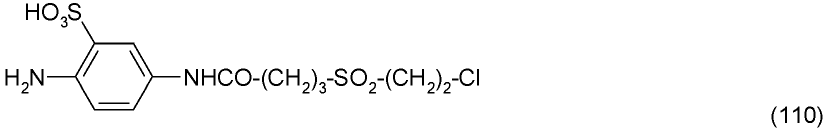 are suspended in 100 ml of water and the volume of the suspension is adjusted to 125 ml by further addition of water. 8.8 g of sodium chloride are added and the mixture is acidified with 5.4 ml of hydrochloric acid (32%). At room temperature, there are added to the resulting solution within 45 minutes 6.2 ml of a 4N sodium nitrite solution. The suspension obtained is stirred until the reaction is completed and excess of nitrite is destroyed by the addition of sulfamic acid.
(b) Second coupling: 183 g of the suspension obtained according to step (a) above are added to 193 g of an aqueous solution containing approximately 12.2 g of the compound of formula (104), and the mixture is adjusted to pH 7.5 by addition of aqueous soda (20% solution). After completion of the coupling reaction, the reaction mixture is adjusted to pH 5.5 by means of hydrochloric acid (32%). Subsequently, the dye solution is freed of salt by dialysis and concentrated by evaporation *in vacuo* to obtain 23.7 g of a product which contains mainly a compound that in the form of the free acid corresponds to the formula 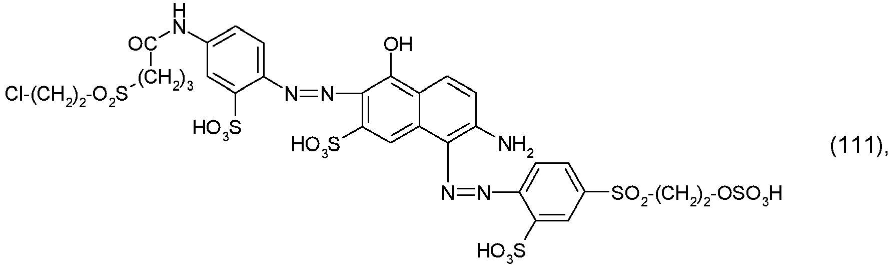 (λₘₐₓ: 522 nm) and dyes cellulose in a red shade with good allround properties.

Example 9 to 35: Following a procedure analogous to that described in one of Examples 4 to 8, but using for the first coupling reaction an equimolar amount of the amine of formula D_{2xy}-NH₂, and for the second coupling reaction an equimolar amount of another amine of formula D_{1xy}-NH₂, and, as the case may be, 2-methylamino-5-naphthol-7-sulfonic acid or 2-sulfomethylamino-5-naphthol-7-sulfonic acid instead of 2-amino-5-naphthol-7-sulfonic acid, there is obtained a compound that in the form of the free acid corresponds to the generic formula (112), wherein D_{1xy}, D_{2xy} and R₂ have the meanings as indicated in Tables 1, 2 and 3.

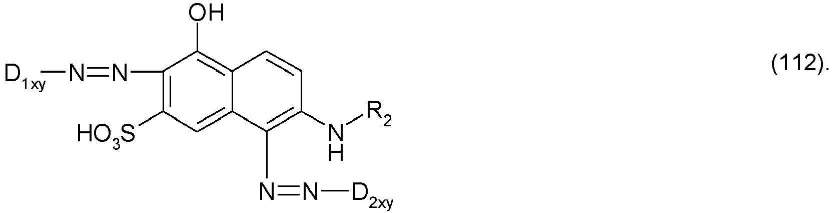

**Table 1:**

| Amine D_{2xy}-NH₂ | D_{2xy} |
|---|---|
| D₂₁₁-NH₂ | 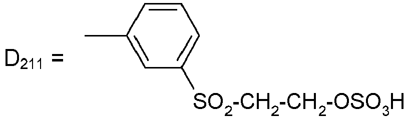 |
| D₂₁₂-NH₂ | 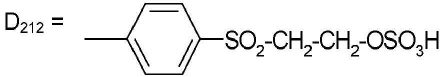 |
| D₂₁₃-NH₂ | 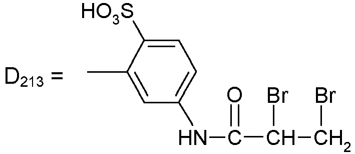 |
| D₂₁₄-NH₂ | 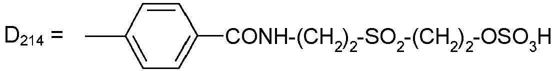 |
| D₂₁₅-NH₂ | 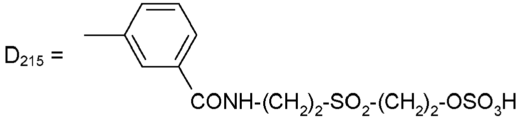 |
| D₂₁₆-NH₂ | 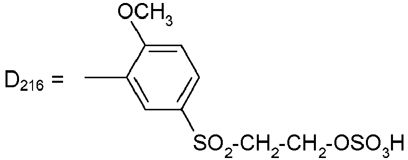 |
| D₂₁₇-NH₂ | 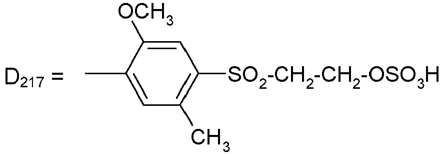 |
| D₂₁₈-NH₂ | 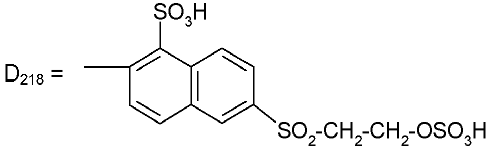 |
| D₂₁₉-NH₂ | 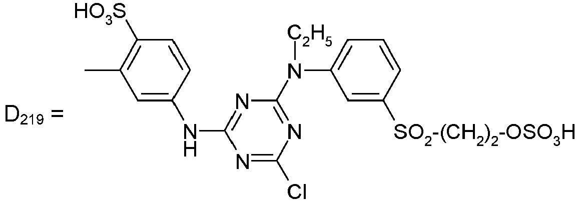 |
| D₂₂₀-NH₂ | 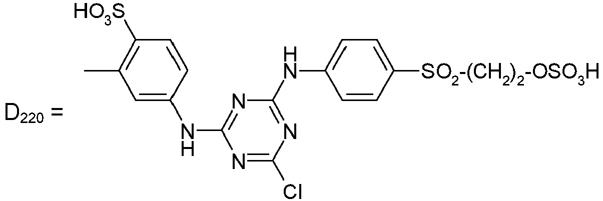 |
| D₂₂₁-NH₂ | 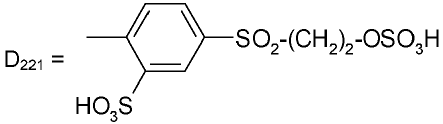 |

**Table 2:**

| Amine D_{1xy}-NH₂ | D_{1xy} |
|---|---|
| D₁₁₁-NH₂ | 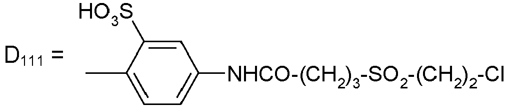 |
| D₁₁₂-NH₂ | 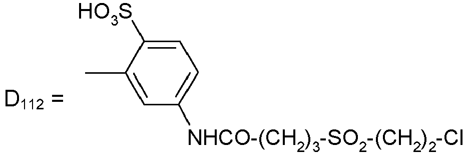 |
| D₁₁₃-NH₂ | 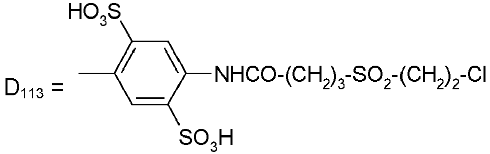 |
| D₁₁₄-NH₂ | 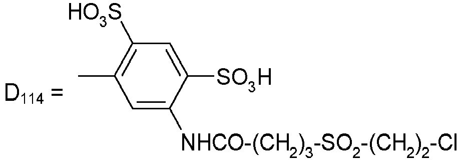 |
| D₁₁₅-NH₂ | 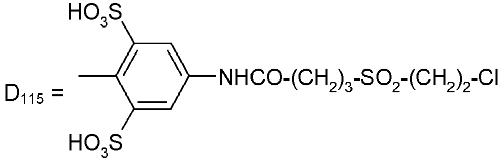 |

**Table 3:**

| Ex | D_{2xy} | D_{1xy} | R₂ | λₘₐₓ in nm |
|---|---|---|---|---|
| 9 | D₂₁₁ | D₁₁₃ | H | 500 |
| 10 | D₂₁₁ | D₁₁₄ | H | 488 |
| 11 | D₂₁₁ | D₁₁₅ | H | 476 |
| 12 | D₂₁₂ | D₁₁₃ | H | 504 |
| 13 | D₂₁₂ | D₁₁₅ | H | 480 |
| 14 | D₂₁₃ | D₁₁₁ | H | 516 |
| 15 | D₂₁₃ | D₁₁₂ | H | 500 |
| 16 | D₂₁₃ | D₁₁₅ | H | 485 |
| 17 | D₂₁₄ | D₁₁₃ | H | 502 |
| 18 | D₂₁₄ | D₁₁₅ | H | 479 |
| 19 | D₂₁₅ | D₁₁₃ | H | 501 |
| 20 | D₂₁₅ | D₁₁₅ | H | 478 |
| 21 | D₂₁₆ | D₁₁₃ | H | 510 |
| 22 | D₂₁₆ | D₁₁₄ | H | 499 |
| 23 | D₂₁₇ | D₁₁₃ | H | 514 |
| 24 | D₂₁₇ | D₁₁₄ | H | 502 |
| 25 | D₂₁₇ | D₁₁₂ | H | 507 |
| 26 | D₂₁₈ | D₁₁₂ | H | 491 |
| 27 | D₂₁₈ | D₁₁₃ | H | 499 |
| 28 | D₂₁₉ | D₁₁₂ | H | 497 |
| 29 | D₂₁₉ | D₁₁₃ | H | 505 |
| 30 | D₂₁₉ | D₁₁₁ | H | 513 |
| 31 | D₂₂₀ | D₁₁₂ | H | 497 |
| 32 | D₂₂₀ | D₁₁₃ | H | 505 |
| 33 | D₂₂₀ | D₁₁₃ | -CH₃ | 523 |
| 34 | D₂₂₁ | D₁₁₂ | -CH₃ | 524 |
| 35 | D₂₂₁ | D₁₁₂ | -CH₂-SO₃H | 507 |

Exhaust dyeing: A cotton tricot fabric is introduced at 60°C into an aqueous dyebath with a liquor ratio of 1:10 which contains the amount of either the dyestuff of formula (108) prepared according to Example 6, or of the reference dyestuff of formula 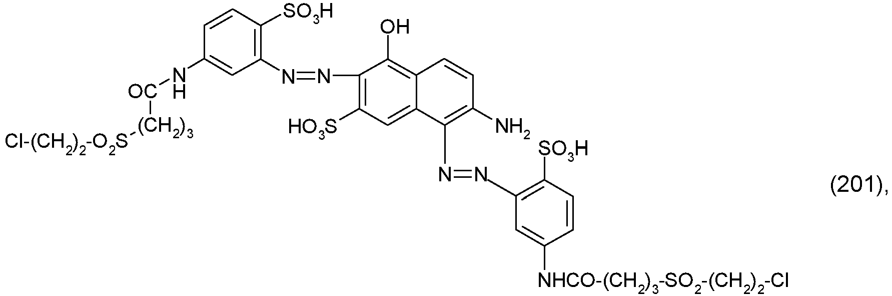 and sodium chloride as indicated in Table 4.

**Table 4: Composition of dyebaths 1 to 6 containing either the dyestuff of Example 6 or the dyestuff of formula (201) as a reference**

| dyebath | 1 | 2 | 3 | 4 | 5 | 6 |
|---|---|---|---|---|---|---|
| % of dyestuff | 0.5 | 1.0 | 2.0 | 4.0 | 6.0 | 8.0 |
| sodium chloride [g/l] | 40 | 50 | 60 | 90 | 100 | 100 |
| sodium carbonate [g/l] | 10 | 12 | 14 | 18 | 18 | 18 |

| | | | | | | |
|---|---|---|---|---|---|---|
| * % of dyestuff is with respect to the weight of the cotton fabric | | | | | | |

After 45 minutes at 60°C calcined sodium carbonate is added in the amount given in Table 4. Dyeing is continued for 45 minutes. The dyed goods are then rinsed with water, soaped and rinsed again and then dried.

The tinctorial strengths of the dyeings obtained with the dyeing liquors 1 to 6 are measured photospectrometrically. Table 5 shows build-up properties of the dyestuff prepared according to Example 6 and the reference dyestuff of formula (201). The dyestuff according to the present invention shows very good build-up properties.

**Table 5: Build-up: dependence of reference depth (RD) from dyestuff concentration**

| | | | | | | | |
|---|---|---|---|---|---|---|---|
| % of dyestuff | 0 | 0.5 | 1.0 | 2.0 | 4.0 | 6.0 | 8.0 |
| RD dyestuff of Ex 6 | 0 | 0.65 | 1.22 | 2.25 | 3.80 | 4.30 | 4.70 |
| RD reference dyestuff | 0 | 0.49 | 0.90 | 1.74 | 3.00 | 3.60 | 3.60 |

Bleached cotton tricot fabrics dyed according to the above method either with 0.8% of the dyestuff of formula (108) (reference depth: 0.92) or with 1.8% of the reference dyestuff of formula (201) (reference depth: 0.96) are subjected to colour fastness test (Xenon arc fading test: 72 h of irradiation). Changes in colour are determined according to the Grey Scale. The fabric dyed with the dyestuff of formula (108) according to the present invention is rated 4-5, whereas the fabric dyed with the reference dyestuff of formula (201) is rated only 3-4.

### Dyeing procedure I

100 parts of cotton fabric are introduced at 60°C into 1500 parts of a dye bath containing 45 g/l of sodium chloride and 2 parts of the reactive dye obtained according to Example 6. After 45 minutes at 60°C, 20 g/l of anhydrous sodium carbonate are added. Dyeing is continued for a further 45 minutes at that temperature. The dyed goods are then rinsed, soaped at the boil for a quarter of an hour with a non-ionic detergent, rinsed again and dried.

As an alternative to the above procedure, the dyeing can be carried out at 80°C instead of at 60°C.

### Dyeing procedure II

0.1 part of the dye according to Example 6 is dissolved in 200 parts of water, and 0.5 part of sodium sulfate, 0.1 part of a levelling adjuvant (based on the condensation product of a higher aliphatic amine and ethylene oxide) and also 0.5 part of sodium acetate are added. The pH is then adjusted to a value of 5.5 using acetic acid (80%). The dye bath is heated at 50°C for 10 minutes and then 10 parts of a woollen fabric are added. Heating is then carried out, over the course of approximately 50 minutes, to a temperature of 100°C and dyeing is carried out at that temperature for 60 minutes, after which the dye bath is allowed to cool to 90°C and the dyed goods are removed. The woollen fabric is washed with hot and cold water, and is then spun and dried.

### Printing procedure I

3 parts of the dye obtained according to Example 6 are sprinkled, with rapid stirring, into 100 parts of a stock thickener containing 50 parts of 5% sodium alginate thickener, 27.8 parts of water, 20 parts of urea, 1 part of sodium m-nitrobenzenesulfonate and 1.2 parts of sodium hydrogen carbonate. The print paste so obtained is used to print a cotton fabric, and the resulting printed material is dried and steamed in saturated steam for 2 minutes at 102°C. The printed fabric is then rinsed, if desired soaped at the boil and rinsed again, and subsequently dried.

### Printing procedure II

(a) Mercerised cotton-satin is padded using a liquor containing 30 g/l of sodium carbonate and 50 g/l of urea (liquor uptake 70%) and dried.
(b) The cotton-satin pre-treated according to Step (a) is printed using an aqueous ink containing
   - 15 % by weight of the reactive dye of formula (108) according to Example 6
   - 15 % by weight of 1,2-propylene glycol and
   - 70 % by weight of water
   using a drop-on-demand inkjet head (bubble jet). The print is dried completely and fixed in saturated steam at 102°C for 8 minutes, cold-rinsed, washed off at the boil, rinsed again and dried.

## Claims

1. A reactive dye of the formula 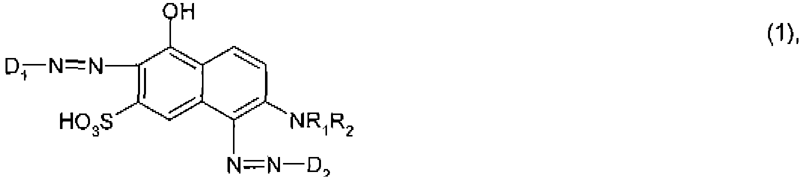 wherein
R₁ is hydrogen,
R₂ is hydrogen, methyl or a radical of the formula -CH₂-SO₃H,
D₁ corresponds to a radical of the formula 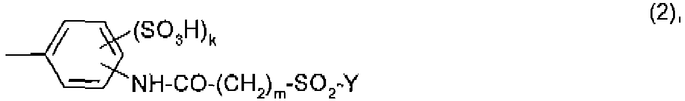 and
D₂ is a radical of formula 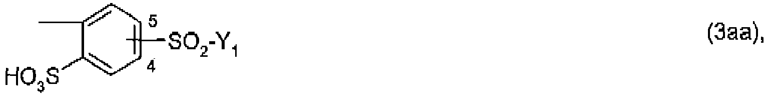
wherein
Y₁ is vinyl or β-sulfatoethyl.
Y is vinyl, β-chloroethyl or β-sulfatoethyl,
k is the number 1 or 2, and
m is the number 2 or 3,

2. A process for the preparation of a dye of the formula (1) according to claim 1, which comprises
(i) diazotisation of approximately one molar equivalent of an amine of the formula
D₂-NH₂ (6)
in customary manner and reaction with approximately one molar equivalent of a compound of the formula 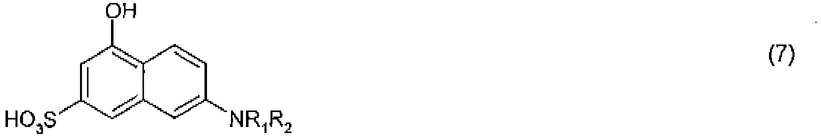 to form a compound of the formula 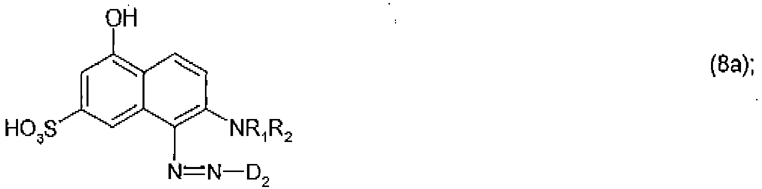 and
(ii) diazotisation of approximately one molar equivalent of an amine of the formula
D₁-NH_{z} (9)
in customary manner and reaction with approximately one molar equivalent of the compound of formula (8a) obtained according to (i) to form a compound of the formula (1) according to claim 1, wherein D₁, D₂, R₁ and R₂ each have the definitions and preferred meanings given in claim 1.

3. The use of a reactive dye according to claim 1 or a reactive dye prepared according to claim 2 in the dyeing or printing of hydroxy-group-containing or nitrogen-containing fibre material.

4. Use according to claim 3, wherein cellulosic fibre material, especially cotton-containing fibre material, is dyed or printed.

5. An aqueous ink that comprises a reactive dye of the formula (1) according to claim 1.

6. A process for printing textile fibre material, paper or plastics film according to the inkjet printing method, which comprises using an aqueous ink according to claim 5.

## Patentansprüche

1. Reaktivfarbstoff der Formel 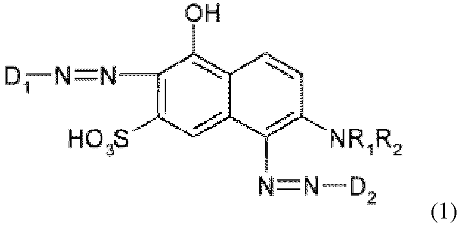 wobei
R₁ Wasserstoff ist,
R₂ Wasserstoff, Methyl oder ein Rest der Formel -CH₂-SO₃H ist,
D₁ einem Rest der Formel 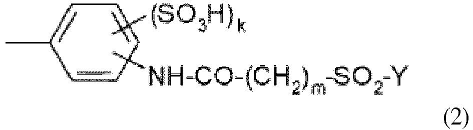
entspricht und
D₂ ein Rest der Formel 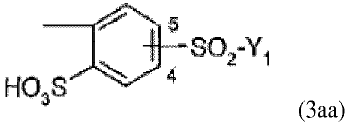 ist,
wobei
Y₁ Vinyl oder β-Sulfatoethyl ist,
Y Vinyl, β-Chloroethyl, oder β-Sulfatoethyl ist,
k die Zahl 1 oder 2 ist und
m die Zahl 2 oder 3 ist.

2. Verfahren zur Herstellung eines Farbstoffs nach Formel (1) gemäß Anspruch 1, das umfasst
(i) Diazotierung von ungefähr einem Moläquivalent eines Amins der Formel
D₂-NH₂ (6)
in üblicher Weise und Reaktion mit ungefähr einem Moläquivalent einer Verbindung der Formel 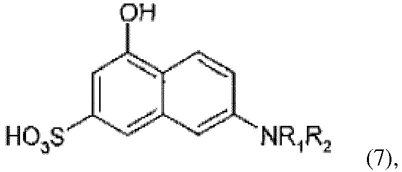 um eine Verbindung der Formel 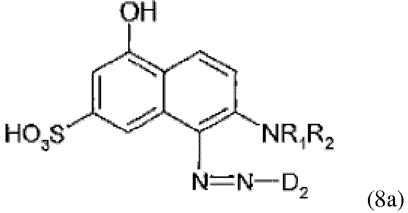 zu bilden und
(ii) Diazotierung von ungefähr einem Moläquivalent eines Amins der Formel
D₁-NH₂ (9)
in üblicher Weise und Reaktion mit ungefähr einem Moläquivalent der Verbindung der Formel (8a), die gemäß (i) erhalten wurde, um eine Verbindung der Formel (1) gemäß Anspruch 1 zu bilden, wobei D₁, D₂, R₁ und R₂ jeweils die Definitionen und bevorzugten Bedeutungen haben, die in Anspruch 1 angegeben sind.

3. Verwendung eines Reaktivfarbstoffs gemäß Anspruch 1 oder eines Reaktivfarbstoffs, der gemäß Anspruch 2 hergestellt wurde, zum Färben oder Bedrucken von Fasermaterial, das Hydroxygruppen oder Stickstoff enthält.

4. Verwendung gemäß Anspruch 3, wobei zellulosehaltiges Fasermaterial, insbesondere Baumwolle-enthaltendes Fasermaterial, gefärbt oder bedruckt wird.

5. Wässrige Tinte, die einen Reaktivfarbstoff der Formel (1) gemäß Anspruch 1 umfasst.

6. Verfahren zum Bedrucken von textilem Fasermaterial, Papier oder Kunststofffilmen gemäß dem Tintenstrahldruckverfahren, das die Verwendung einer wässrigen Tinte gemäß Anspruch 5 umfasst.

## Revendications

1. Colorant réactif de la formule : 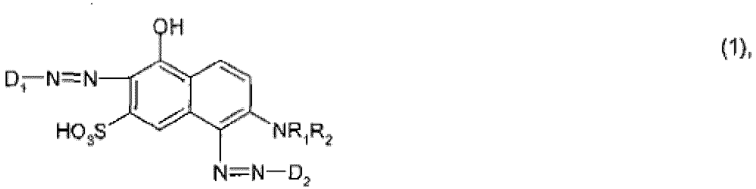 dans laquelle :
R₁ représente hydrogène ;
R₂ représente hydrogène, méthyle ou un radical de la formule -CH₂-SO₃H ;
D₁ correspond à un radical de la formule : 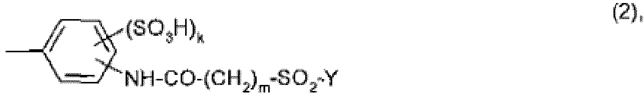 et
D₂ est un radical de la formule : 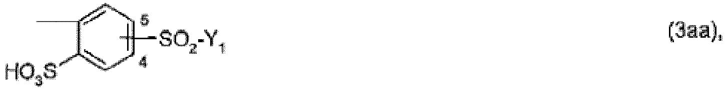
où
Y₁ représente vinyle ou β-sulfatoéthyle ;
Y représente vinyle, β-chloroéthyle ou β-sulfatoéthyle ;
k est le nombre 1 ou 2 ; et
m est le nombre 2 ou 3.

2. Procédé de préparation d'un colorant de la formule (1) selon la revendication 1, qui comprend :
(i) la diazotation d'approximativement un équivalent molaire d'une amine de la formule :
D₂-NH₂ (6)
d'une manière habituelle et la réaction avec approximativement un équivalent molaire d'un composé de la formule : 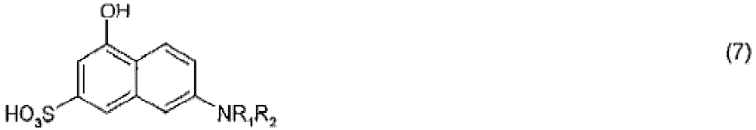 pour former un composé de la formule : 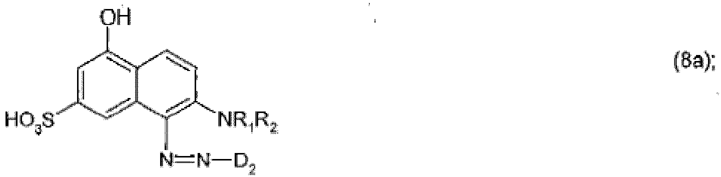 et
(ii) la diazotation d'approximativement un équivalent molaire d'une amine de la formule :
D₁-NH₂ (9)
d'une manière habituelle et la réaction avec approximativement un équivalent molaire du composé de formule (8a) obtenu conformément à (i) pour former un composé de la formule (1) selon la revendication 1, où D₁, D₂, R₁ et R₂ ont chacun les définitions et les significations préférées données dans la revendication 1.

3. Utilisation d'un colorant réactif selon la revendication 1 ou d'un colorant réactif préparé selon la revendication 2 dans la teinture ou l'impression d'une matière fibreuse contenant des groupes hydroxy ou contenant de l'azote.

4. Utilisation selon la revendication 3, dans laquelle une matière fibreuse cellulosigue, notamment une matière fibreuse contenant du coton, est teinte ou imprimée.

5. Encre aqueuse qui comprend un colorant réactif de la formule (1) selon la revendication 1.

6. Procédé d'impression d'une matière fibreuse textile, d'un papier ou d'un film de matière plastique conformément à la méthode d'impression à jet d'encre, qui comprend l'utilisation d'une encre aqueuse selon la revendication 5.
